# EUROPÄISCHE PATENTANMELDUNG

(11) **EP 0 680 942 A1**
(43) Veröffentlichungstag der Anmeldung: **08.11.1995**
(21) Anmeldenummer: 95106749.5
(22) Anmeldetag: 04.05.1995
(51) Int. Cl.: C07C 11/02, C07C 2/30, C07C 2/22

(54) **Verfahren zur Oligomerisierung von Alpha-Olefinen zu Poly-Alpha-Olefinen**

(30) Priorität: 05.05.1994 DE 4415912
(71) Anmelder: Linde Aktiengesellschaft, D-65189 Wiesbaden (DE)
(72) Erfinder: Moussalli, George, Dr.-Ing., D-82166 Gräfelfing (DE); Fritz, Peter, Dr. rer. nat., D-82008 Unterhaching (DE); Bölt, Heinz, Dipl.-Ing., D-82515 Wolfratshausen (DE); Matkovskii, Peter Evgenievich, Prof. Dr., Noginsk District, Moscow region, 142432 (RU); Chekry, Pavel Semenovich, Dr., Deceased (RU); Melnikov, Valeri Nicolaevich, Dr., Novokuibyshevsk, Samara Region, 446206 (RU)
(74) Vertreter: Kasseckert, Rainer

(57) **Zusammenfassung**

Ein Verfahren zur Oligomerisierung von Alpha-Olefinen zu Poly-Alpha-Olefinen in Anwesenheit eines homogenen Flüssig-Katalysators, wobei die Alpha-Olefine auch als Lösungsmittel für den Flüssig-Katalysator dienen und die Oligomerisierung unter Druck stattfindet, indem die Alpha-Olefine und der Flüssig-Katalysator als Lösung einen Reaktor durchlaufen, an dessen Auslaßende ein aus verschieden hoch oligomerisierten Alpha-Olefinen und nicht umgesetzten Alpha-Olefinen bestehendes Gemisch abfließt, das durch Destillation fraktioniert wird, wobei die nicht umgesetzten Monomere zusammen mit einem Teil der Di- und Trimeren sowie weiteren leichten Alpha-Olefinen mit weniger als 20 C-Atomen vor den Reaktor zurückgeführt und mit den Alpha-Olefinen vermischt werden, während die höheren Oligomere als Produkt gewonnen werden.

## Beschreibung

Die Erfindung betrifft ein Verfahren zur Oligomerisierung von Alpha-Olefinen zu Poly-Alpha-Olefinen in Anwesenheit eines homogenen Flüssig-Katalysators, wobei die Alpha-Olefine auch als Lösungsmittel für den Flüssig-Katalysator dienen und die Oligomerisierung unter Druck stattfindet, indem die Alpha-Olefine und der Flüssig-Katalysator als Lösung einen Reaktor durchlaufen, an dessen Auslaßende ein aus verschieden hoch oligomerisierten Alpha-Olefinen und nicht umgesetzten Alpha-Olefinen bestehendes Gemisch abfließt, das durch Destillation fraktioniert wird.

Poly-Alpha-Olefine bilden die Hauptkomponenten zur Herstellung synthetischer Öle, z.B. Motoröle, Getriebeöle, Kabelöle und Isolieröle. Sie haben deshalb eine erhebliche wirtschaftliche Bedeutung.

Aus der GB-PS 1,535,325 ist ein Verfahren für die Oligomierisierung von Alpha-Olefinen bekannt. Bei dem bekannten Verfahren wird von Alpha-Olefinen ausgegangen, die mindestens drei C-Atome haben. Sie werden zu Oligomeren mit 20 bis 60 C-Atomen polymerisiert. Zu diesem Zweck werden bei dem bekannten Verfahren Lösungen der beiden Katalysator-Komponenten bereitet, wobei das Lösungsmittel entweder das umzusetzende Monomer oder ein anderer Kohlenwasserstoff sein kann, und die beiden Lösungen in einem Reaktor mit flüssigem Monomer zusammengebracht. Als Katalysator-Komponenten werden Aluminiumalkylhalogenide und organische Halogenide vorgeschlagen.Das Reaktionsgemisch wird sodann gerührt und die entstandenen Reaktionsprodukte teils durch Gas-Flüssigkeits-Chromatographie bestimmt, teils durch Destillation getrennt.

Bei dem bekannten Verfahren ist nicht angegeben, wie mit dem nicht umgesetzten Monomer bzw. mit Oligomeren, die zu niedrige Siedepunkte und zu niedrige Viskosität besitzen, umgegangen werden soll. Das bekannte Verfahren ist deswegen verbesserungsbedürftig.

Es ist die Aufgabe der vorliegenden Erfindung, das bekannte Verfahren hinsichtlich seiner Ausbeute an höheren Oligomeren zu verbessern.

Diese Aufgabe wird erfindungsgemäß dadurch gelöst, daß die nicht umgesetzten Monomere zusammen mit einem Teil der Di- und Trimeren sowie weiteren leichten Alpha-Olefinen mit weniger als 20 C-Atomen vor den Reaktor zurückgeführt und mit den Alpha-Olefinen vermischt werden, während die höheren Oligomere als Produkt gewonnen werden.

Beim erfindungsgemäßen Verfahren werden zunächst die Reaktionsteilnehmer, d.h. ein Alpha-Olefin oder Alpha-Olefin-Gemisch, vorzugsweise 1-Buten und 1-Hexen, mit den Katalysatoren in Lösung vermischt und einem Oligomerisierungs-Reaktor zugeführt.

Als für die Zwecke der Erfindung geeignete Katalysator-Komponenten haben sich Ethylaluminiumsequichlorid als die eine Komponente und Benzylchlorid als die andere Komponente erwiesen. Beide Verbindungen sind flüssig. Sie werden vorzugsweise in unverdünnter Form eingesetzt, können jedoch auch mit einem Lösungsmittel, z.B. 1-Hexen, verdünnt sein.

Als weitere geeignete, wenngleich weniger bevorzugte Katalaysatoren haben sich folgende erwiesen:
Ethylaluminiumdichlorid, Diethylaluminiumchlorid, Aluminiumtrichlorid und tert. Butylchlorid.

Zweckmäßigerweise werden die Katalysatoren und die Ausgangs-Alpha-Olefine vor dem Reaktor gekühlt, damit die Temperatur bei der Oligomerisierung, die exotherm ist, nicht zu stark ansteigt. Außerdem braucht der Reaktor dann weniger gekühlt zu werden. In manchen Fällen kann es sich als zweckmäßig erweisen, hinter dem ersten Oligomerisierungs-Reaktor noch einen zweiten zu installieren, um die Reaktion zu vervollständigen.

Das Oligomerisierungs-Produkt wird nach dem oder den Reaktoren über einen Adsorber geschickt, der beispielsweise mit Silikagel oder Aluminiumoxidgel ausgestattet ist, um die Katalysator-Komponenten aus dem Reaktionsgemisch zu entfernen. Nach Passieren des Adsorbers wird das Oligomerengemisch in einen Fallfilmverdampfer geleitet. In diesem Fallfilmverdampfer erfolgt eine Trennung der C₄/C₆-Kohlenwasserstoffe von den Oligomeren. Erfindungsgemäß werden die C₄/C₆-Kohlenwasserstoffe wieder vor den ersten Reaktor zurückgeführt und dem Einsatzkohlenwasserstoff beigemischt.

Anstelle des Fallfilmverdampfers kann an dieser Stelle auch ein Dünnschichtverdampfer oder eine mit Einbauten ausgestattete Rektifiziersäule eingesetzt werden. Für den angestrebten Zweck, nämlich eine grobe Trennung eines Kohlenwasserstoffgemisches, wird jedoch die wesentlich einfachere und billigere Konstruktion eines Fallfilmverdampfers vorgezogen.

Die flüssig aus dem Fallfilmverdampfer ablaufenden Oligomere, schon relativ hochsiedene Gebilde, werden, zum Zwecke der Siedepunktserniedrigung unter vermindertem Druck, gegebenenfalls unter Vakuum, in eine Fraktion bis etwa C₂₀ und in eine C(₂₀₊)-Fraktion von Poly-Alpha-Olefinen getrennt. Die C(₂₀₋)-Fraktion, die also die Kohlenwasserstoffe mit 8 bis 20 Kohlenstoffatomen enthält, wird gasförmig aus dem Kopf der Trennsäule abgezogen und zur Verflüssigung abgekühlt. Sie kann als Produkt abgegeben werden, es besteht jedoch auch, je nach deren Zusammensetzung und in Abhängigkeit von der Zusammensetzung des Ausgangskohlenwasserstoff-Gemisches, erfindungsgemäß die Möglichkeit, diese Fraktion, genauso wie die oben erwähnte C₄/C₆-Kohlenwasserstoff-Fraktion, wieder dem Ausgangsgemisch zuzusetzen und einer erneuten Oligomerisierung zu unterwerfen.

Die vom Fuß der Trennsäule abfließenden Poly-Alpha-Olefine werden dann nochmals in einer weiteren Trennsäule in eine schwerere und in eine leichtere Fraktion zerlegt und getrennt verschiedenen Verwendungszwecken zugeführt.

Beim erfindungsgemäßen Verfahren hat es sich als zweckmäßig erwiesen, die Oligomerisierungsreaktion in einem Temperaturbereich von 5 bis 150°C und in einem Druckbereich von 10 bis 50 bar ablaufen zu lassen.

Ein Über- oder Unterschreiten der Temperatur- und Druckgrenzen hat folgende Nachteile:
Niedrigere Drücke und/oder höhere Temperaturen haben eine unerwünschte Gasphasenbildung bei der Oligomerisierung zur Folge, während bei niedrigeren Temperaturen die Reaktionen zu langsam ablaufen.

Die erfindungsgemäße Rückführung der nicht umgesetzten Monomere zusammen mit niedrigen Oligomerisaten hat den Vorteil einer erheblichen Ausbeuteverbesserung und damit Einsparung von Rohprodukt. Während bei dem bekannten Verfahren der GB-PS 1,535,325 im Pilotversuch (Beispiel VI) lediglich eine Ausbeute von 56% (C₂₄ bis C₅₆) erzielt wurde, gestattet die vorliegende Erfindung im industriellen Maßstab eine Ausbeutesteigerung auf über 80% an höheren Oligomeren, wie im nachfolgenden noch anhand eines speziellen Ausführungsbeispiels belegt werden wird.

Ferner können durch die erfindungsgemäße Rückführung bestimmter Monomere und/oder Oligomere auch die Produkteigenschaften den jeweiligen Erfordernissen angepaßt werden.

Die Erfindung sei nunmehr anhand eines in einer Figur schematisch dargestellten Ausführungsbeispiels noch näher erläutert.

Durch Leitung 1 werden der Anlage als Einsatzmaterialien 250 kg/h 1-Buten und 250 kg/h 1-Hexen zugeführt. Das Gemisch hat eine Temperatur von 5°C. Bevor es in den Reaktor 2 eintritt, werden ihm aus dem schematisch dargestellten Vorratsbehälter 3 10 kg/h Benzylchlorid zugemischt. In den Kopf des Reaktors werden außerdem aus dem schematisch dargestellten Vorratsbehälter 4 10 kg/h Ethylaluminiumsesquichlorid aufgegeben.

Der Reaktor 2 ist ein Röhrenreaktor, wobei die Oligomerisierungs-Reaktion innerhalb der Röhren stattfindet. Der Raum außerhalb der Röhren ist von einem Kühlmittel umgeben, das dazu dient, die exotherme Reaktionswärme abzuführen, und dessen Zu- und Ablauf nur schematisch dargestellt ist. Nach Passieren des Reaktors 2 gelangt das Oligomeren-Gemisch durch eine Leitung 5 in einen zweiten Oligomerisierungs-Reaktor 6, der als Leerrohrreaktor ausgebildet ist und wo die Oligomerisierungs-Reaktionen zu Ende ablaufen. Flash-Gase aus diesem Reaktor werden im Kühler 7 gekühlt und wieder in den Reaktor 6 zurückgeführt.

Die Reaktionsprodukte werden im Kühler 8 auf eine Temperatur von 80°C abgekühlt und von unten in einen Adsorber 9 eingeführt. Dieser Adsorber 9 ist mit einer Packung aus Aluminiumoxidgel oder Silikagel versehen, die die Aufgabe hat, die Katalysator-Komponten aus dem Reaktionsgemisch adsorptiv zu entfernen. In der schematischen Darstellung ist der Adsorber 9 aus Gründen der Übersichtlichkeit lediglich einfach dargestellt. In Wirklichkeit ist er doppelt ausgeführt, so daß jeweils einer der beiden Adsorber auf eine beliebige, im Stand der Technik bekannte Weise regeneriert werden kann.

Das von den Katalysator-Komponenten befreite Poly-Alpha-Olefin-Gemisch wird durch Leitung 10 in einen Fallfilmverdampfer 11 überführt. Der Fallfilmverdampfer 11 ist mit einem Heizmantel ausgestattet, durch den ständig Heizmittel fließt, dessen Zu- und Ablauf lediglich schematisch dargestellt ist. Im Fallfilmverdampfer 11, der auf einer Temperatur von 200°C gehalten wird, werden die nicht umgesetzten C₄-bis C₆-Kohlenwasserstoffe von den Poly-Alpha-Olefinen getrennt. Die nicht umgesetzten Kohlenwasserstoffe werden vom Kopf des Fallfilmverdampfers 11 über Leitung 12 abgezogen, im Kühler 13 durch Abkühlung auf 8°C verflüssigt und im Abscheider 14 aufgefangen. Von dort werden sie mit Hilfe der Pumpe 15 durch Leitung 16 und Leitung 17 wieder zur Leitung 1 zurückgeführt und dort mit dem Ausgangskohlenwasserstoff-Gemisch vereinigt. Der Rücklauf aus dem Abscheider 14 beträgt 400 kg/h.

Die Poly-Alpha-Olefin-Fraktion aus dem Fallfilmverdampfer 11 wird mit Hilfe einer Pumpe 37 durch Leitung 18 in einer Menge von 600 kg/h in den oberen Teil einer Trennsäule 19 eingespeist. Die Trennsäule 19 ist mit Einbauten versehen und mit einer Sumpfheizung (nur schematisch dargestellt) ausgestattet. In der Trennsäule 19 werden die C₈- bis C₂₀-Olefine, die gasförmig vom Kopf der Trennsäule abziehen, von den höheren Oligomerisierungsprodukten getrennt. Da es sich bei den in dieser Säule aufzuarbeitenden Produkten bereits um relativ hochsiedende handelt, wird die Säule bei Unterdruck betrieben, was den Siedepunkt senkt. Zweckmäßig ist hier ein Druck von 0,1 bar. Die Kopf-Fraktion wird im Kühler 20 verflüssigt und im Abscheider 21 aufgefangen. Der Unterdruck in der Trennsäule 19 wird durch die Vakuumpumpe 22 aufrechterhalten. Die Flüssigkeit aus 21 wird mit Pumpe 23 weiterbefördert. Ein Teil der Flüssigkeit wird der Trennsäule 19 durch Leitung 24 wieder als Rücklaufflüssigkeit zugeführt. Der übrige Teil, nämlich 180 kg/h, kann entweder durch Leitung 25 als Produkt abgegeben oder, wenn keine Verwendungsmöglichkeiten für ein solches Produkt bestehen, durch Leitung 26 zum überwiegenden Teil (100 kg/h) zur Leitung 17 geführt und wieder mit dem Ausgangsprodukt vermischt werden.

Das Bodenprodukt der Trennsäule 19 wird mit Hilfe einer Pumpe 27 und Leitung 28 auf den oberen Teil einer Poly-Alpha-Olefin-Trennsäule 29 befördert. Diese Trennsäule ist ebenfalls mit Einbauten versehen und mit einer Sumpfheizung (nur schematisch dargestellt) ausgestattet. Sie wird zweckmäßigerweise ebenfalls bei Unterdruck, vorzugsweise bei 0,02 bar, betrieben.

In der Poly-Alpha-Olefin-Trennsäule 29 erfolgt eine Trennung in leichtere und schwerere Poly-Alpha-Olefine. Der Schnitt liegt etwa zwischen C₃₀ und C₃₆. Die schwereren Poly-Alpha-Olefine werden durch Leitung 30 in einer Menge von 240 kg/h als Produkt abgegeben. Die leichteren Poly-Alpha-Olefine, die gasförmig vom Kopf der Trennsäule 29 abziehen, werden im Kühler 31 verflüssigt und im Abscheider 32 aufgefangen. Der Unterdruck in der Trennsäule 29 wird durch die Vakuumpumpe 33 aufrechterhalten. Der Druck der aus 32 ablaufenden Flüssigkeit wird in der Pumpe 34 auf 4 bar erhöht. Ein Teil der ablaufenden Flüssigkeit, nämlich 180 kg/h, wird durch Leitung 36 als Produkt abgegeben. Der Rest wird der Trennsäule 29 durch Leitung 35 wieder als Rücklauf zugeführt.

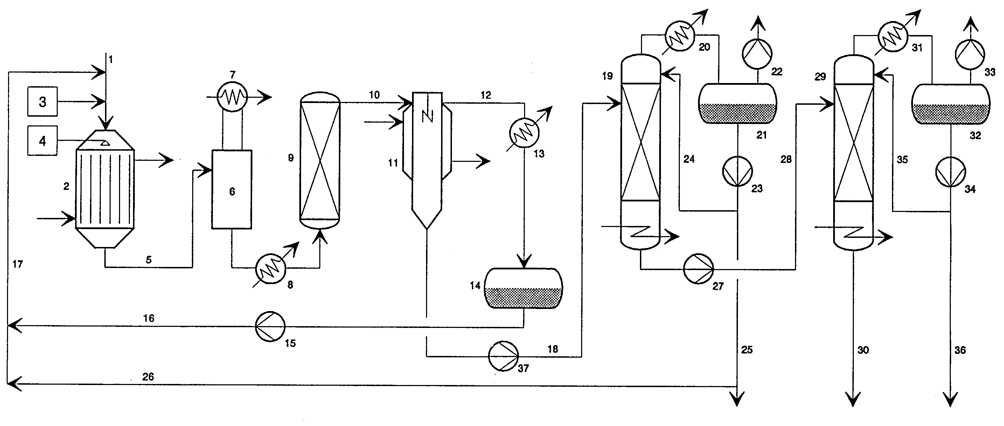

## Patentansprüche

1. Verfahren zur Oligomerisierung von Alpha-Olefinen zu Poly-AlphaOlefinen in Anwesenheit eines homogenen Flüssig-Katalysators, wobei die Alpha-Olefine auch als Lösungsmittel für den Flüssig-Katalysator dienen und die Oligomerisierung unter Druck stattfindet, indem die Alpha-Olefine und der Flüssig-Katalysator als Lösung einen Reaktor durchlaufen, an dessen Auslaßende ein aus verschieden hoch oligomerisierten Alpha-Olefinen und nicht umgesetzten Alpha-Olefinen bestehendes Gemisch abfließt, das durch Destillation fraktioniert wird, **dadurch gekennzeichnet**, daß die nicht umgesetzten Monomere zusammen mit einem Teil der Di- und Trimeren sowie weiteren leichten Alpha-Olefinen mit weniger als 20 C-Atomen vor den Reaktor zurückgeführt und mit den Alpha-Olefinen vermischt werden, während die höheren Oligomere als Produkt gewonnen werden.

2. Verfahren nach Anspruch 1, **dadurch gekennzeichnet**, daß die Monomeren Alpha-Olefine C₃- bis C₁₈- Kohlenwasserstoffe umfassen.

3. Verfahren nach Anspruch 1, **dadurch gekennzeichnet**, daß der Flüssig-Katalysator aus Ethylaluminiumsesquichlorid und Benzylchlorid besteht.

4. Verfahren nach Anspruch 1, **dadurch gekennzeichnet**, daß der Flüssig-Katalysator aus der Gruppe, bestehend aus Ethylaluminiumdichlorid, Diethylaluminiumchlorid, Aluminiumtrichlorid und tert. Butylchlorid ausgewählt ist.

5. Verfahren nach den vorhergehenden Ansprüchen, **dadurch gekennzeichnet**, daß es bei einer Temperatur zwischen 5 und 150 °C durchgeführt wird.

6. Verfahren nach den vorhergehenden Ansprüchen, **dadurch gekennzeichnet**, daß es bei einem Druck zwischen 10 und 50 bar durchgeführt wird.
